# EUROPEAN PATENT APPLICATION

(11) **EP 4 775 221 A1**
(43) Date of publication of application: **15.07.2026**
(21) Application number: 23951114.0
(22) Date of filing: 05.09.2023
(51) Int. Cl.: A61K 39/02, A61K 39/116, C07K 14/30, C07K 19/00, C12N 15/62, C12N 15/31, C12N 15/10, A61P 11/00, A61P 29/00, A61P 31/04, C12R 1/35

(54) **COMPOSITION FOR PREVENTING AND TREATING MYCOPLASMA HYORHINIS INFECTION, AND PREPARATION METHOD THEREFOR**

(71) Applicant: Agricultural Technology Research Institute, Hsinchu City 300 (TW)
(72) Inventor: LIN, Jiunn-Horng, Hsinchu City 300 (TW); CHEN, Zeng-Weng, Hsinchu City 300 (TW); WANG, Jyh-Perng, Hsinchu City 300 (TW); CHEN, Ching-I, Hsinchu City 300 (TW); HUANG, Wen-Zheng, Hsinchu City 300 (TW); LIN, Hui-Jie, Hsinchu City 300 (CN); PENG, Tzu-Ting, Hsinchu City 300 (TW); HSUAN, Shih-Ling, Hsinchu City 300 (TW)
(74) Representative: Charrier Rapp & Liebau
(86) International application number: PCT/CN2023/117008
(87) International publication number: WO 2025/050285

(57) **Abstract**

The present application provides a composition for preventing and treating *M*. *hyorhinis* infection, which uses a fusion protein of C-terminus of DnaK - N-terminus of P72 - XylF as the active ingredient. The present application further provides an expression vector and method for producing the aforementioned fusion protein. According to the disclosure of the present application, the purpose of avoiding the symptoms caused by *M. hyorhinis* infection can be achieved, and the cost of producing the composition for preventing and treating M. *hyorhinis* infection can be reduced by leveraging the advantage of the fusion protein in containing multiple antigens at once.

## Description

### BACKGROUND OF THE INVENTION

### FIELD OF THE INVENTION

**The** present application relates to a composition for preventing and treating mycoplasma infection and, more particularly, to a composition for preventing and treating *Mycoplasma hyorhinis* (*M. hyorhinis*) infection and a method for producing the same.

### DESCRIPTION OF THE PRIOR ART

*Mycoplasma* spp. are the smallest known prokaryotic microorganisms capable of extracellular replication, ranging in size from spherical with a diameter ranging from 0.3 µm to 0.9 µm to filamentous with a length of up to 1 µm. Nutritionally, *Mycoplasma* spp. are fastidious bacteria, difficult to culture and extremely slow-growing. Several species of *Mycoplasma* are known to be associated with various human and animal diseases.

According to literature reports, several *Mycoplasma* spp. are associated with various human and animal diseases. Among them, *M. hyorhinis* is a respiratory pathogen in pigs, with an infection rate ranging from approximately 25% to 93%. It causes swine enzootic pneumonia (SEP), a highly contagious but low-mortality chronic respiratory disease. *M*. *hyorhinis* is also associated with the occurrence of polyserositis, arthritis, and meningitis.

Pigs infected solely with *M*. *hyorhinis* typically do not die, but infection can lead to reduced feed conversion ratios, stunted growth, inflammatory responses, immunosuppression, and secondary infection by other bacterial or viral pathogens, resulting in significant economic losses for the pig farming industry.

Currently, inactivated *M*. *hyorhinis* vaccines are available for use in the pig farming industry. However, *M. hyorhinis* is difficult to culture, the culture media used are expensive, *M*. *hyorhinis* may pose a risk of inducing human cancer, and the vaccine preparation process requires high-level protective measures. Therefore, developing highly safe and low-cost subunit vaccines is of great significance and demand in this field.

In previous research, the inventors of the present application screened three antigens from 17 antigens that could serve as active ingredients for subunit vaccines, i.e. XylF, DnaK, and P72. However, from a vaccine manufacturing perspective, the production process for multiple antigens presents bottlenecks due to high labor and time costs, and the optimal production conditions for individual antigens, such as bacterial fermentation and protein purification, require processes to be established based on the characteristics of each individual antigen.

### SUMMARY OF THE INVENTION

**To** address the needs in this field, the present application provides a composition for preventing and treating *M*. *hyorhinis* infection. By developing a subunit vaccine containing a fusion protein, the vaccine production process and cost are significantly reduced. Results of swine immunization challenge experiments show that the vaccine prepared with the fusion protein has high safety and can effectively alleviate lesions caused by *M. hyorhinis* infection.

To achieve the above objectives, the present invention provides a composition for preventing and treating *M*. *hyorhinis* infection, which may include: (a) a fusion protein of C-terminus of DnaK - N-terminus of P72 - XylF of the *M*. *hyorhinis;* and (b) a pharmaceutically acceptable adjuvant.

Preferably, a concentration of the fusion protein may be 50 to 500 µg/mL, 60 to 400 µg/mL, 70 to 300 µg/mL, 80 to 200 µg/mL, or 90 to 100 µg/mL. In one embodiment, the concentration of the fusion protein may be 100 µg/mL.

Preferably, in the composition, the C-terminus of DnaK may include a sequence set forth in SEQ ID NO: 9; the N-terminus of P72 may include a sequence set forth in SEQ ID NO: 11; or the XylF may include a sequence set forth in SEQ ID NO: 13.

Preferably, in the composition, the fusion protein may include a sequence set forth in SEQ ID NO: 7.

Preferably, the pharmaceutically acceptable adjuvant for the composition may be a Freund's complete or incomplete adjuvant, an alumina gel, a surfactant, an anionic polymer, a peptide, an oil emulsion or a combination thereof.

Preferably, the composition may further include a pharmaceutically acceptable additive. In one embodiment, the pharmaceutically acceptable additive may include a solvent, a stabilizer, a diluent, a preservative, an antimicrobial agent, an antifungal agent, an isotonic agent, an absorption delaying agent or a combination thereof.

Another object of the present application is to provide an expression vector for expressing the aforementioned fusion protein, which may include: (a) a nucleotide sequence encoding a fusion protein of C-terminus of DnaK - N-terminus of P72 - XyIF; (b) a replicon; and (c) an expression element, which may include a promoter, a terminator and a ribosome binding site.

Preferably, in the expression vector, a nucleotide sequence encoding the C-terminus of DnaK may encode an amino acid sequence set forth in SEQ ID NO: 9; a nucleotide sequence encoding the N-terminus of P72 may encode an amino acid sequence set forth in SEQ ID NO: 11; and a nucleotide sequence encoding the XylF may encode an amino acid sequence set forth in SEQ ID NO: 13.

Preferably, in the expression vector, the nucleotide sequence encoding the C-terminus of DnaK may include a nucleotide sequence set forth in SEQ ID NO: 8 or a reverse complementary sequence thereof; the nucleotide sequence encoding the N-terminus of P72 may include a nucleotide sequence set forth in SEQ ID NO: 10 or a reverse complementary sequence thereof; and the nucleotide sequence encoding the XylF may include a nucleotide sequence set forth in SEQ ID NO: 12 or a reverse complementary sequence thereof.

Preferably, in the expression vector, the nucleotide sequence encoding the fusion protein may encode an amino acid sequence set forth in SEQ ID NO: 7.

Preferably, in the expression vector, the nucleotide sequence encoding the fusion protein may include a nucleotide sequence set forth in SEQ ID NO: 6 or a reverse complementary sequence thereof.

Preferably, in the expression vector, the expression vector may include a nucleotide sequence set forth in SEQ ID NO: 5.

Preferably, in the expression vector, the fusion protein may further include a tag. In one embodiment, the tag may include a histidine tag (His-tag), a glutathione S-transferase tag (GST-tag), a FLAG tag, a maltose binding protein-tag (MBP-tag), a hemagglutinin tag (HA-tag) or a combination thereof.

Preferably, the expression vector may further include a selectable marker. In one embodiment, the selectable marker may include a drug-resistant selectable marker, a non-drug-resistant selectable marker or a combination thereof.

Preferably, in the expression vector, the fusion protein may be expressed in E. *coli.*

Yet another object of the present application is to provide a method for producing a fusion protein of C-terminus of DnaK - N-terminus of P72 - XylF, which may include transcribing the aforementioned nucleotide sequence in an expression system, followed by translation to obtain the fusion protein.

Yet another object of the present application is to provide use of a fusion protein of C-terminus of DnaK - N-terminus of P72 - XyIF in preparation of a composition for preventing and treating symptoms caused by *M. hyorhinis* infection.

Preferably, the symptoms caused by the *M*. *hyorhinis* infection in the use may include pleurisy, peritonitis, pericarditis, arthritis or a combination thereof.

### BRIEF DESCRIPTION OF THE DRAWINGS

Figure 1 shows the construction process of pET29-MHY30.
Figure 2 shows the expression of the fusion protein of C-terminus of DnaK - N-terminus of P72 - XylF in *E*. *coli* by analysis using (A) protein electrophoresis and (B) western blotting. M: PageRuler^{™} Pre-stained Protein Ladder (Thermo Fisher Scientific, USA); C: Control group, total cell lysate of *E*. *coli* BL21(DE3) (pET29a); T: *E. coli* BL21(DE3) (pET29-MHY30) total cell lysate; S: *E*. *coli* BL21 (DE3) (pET29-MHY30) soluble protein.
Figure 3 shows the purification of the fusion protein of C-terminus of DnaK - N-terminus of P72 - XylF.

### DETAILED DESCRIPTION OF THE EMBODIMENTS

Unless otherwise defined, all technical and scientific terms used in the present application have the same meaning as commonly understood by those skilled in the art. In the event of any conflict, the definitions contained herein shall prevail.

When referring to concentration in the present application, the terms "percentage" and "%" may refer to mass concentration or volume percentage concentration. The mass concentration is defined as the percentage of the mass of the solute divided by the volume of the corresponding mixture (including the volume of the solute) and is expressed herein as "percentage" or "%" followed by "(w/v)". The volume percentage concentration is defined as the percentage of the volume of the solute to the total volume of the solution and is expressed herein as "percentage" or "%" followed by "(v/v)".

As used herein, the terms "produced from" and "comprising" are synonymous. As used herein, the terms "includes/including," "comprises/comprising", "has/having", "contains/containing" or any other variation thereof are intended to encompass non-exclusivity. For example, a composition, process, method, article or device containing a plurality of elements listed is not necessarily limited to those listed but may include other elements not expressly listed but inherent to the composition, process, method, article or device. The term "includes/including" is generally used in the sense of comprises/comprising, that is, allowing the presence of one or more other features or components.

**The** indefinite articles "a" and "an" preceding an element or component in the present application are intended to indicate, in a non-restrictive manner, the number of instances (i.e., the number of occurrences) of that element or component. Therefore, "a" or "an" should be understood to include one or at least one, and the singular form of the element or component also includes the plural, unless the number clearly refers to the singular.

The present application provides numerous sequences, including primer sequences, nucleotide sequences, and amino acid sequences. These sequences should be allowed to have slight mutations, provided their function and effect in an organism remain unchanged. That is, even if the sequences differ, if they produce the same function and effect as the sequences provided in the present application, they should be considered the same sequence. For example, in primer sequence analysis, primers that exhibit minor sequence variations but amplify the same product should be regarded as equivalent. At the nucleotide sequence level, when a silent mutation is present in a gene, the resulting transcribed amino acid sequence remains identical to that prior to the mutation. Therefore, the sequences before and after the mutation should be regarded as equivalent. Similarly, at the amino acid sequence level, amino acid bases that have similar physical properties (e.g., identical charge, similar hydrophilicity or hydrophobicity, or the presence of aromatic rings), mutate with each other but do not alter the original structure or function should be considered equivalent sequences. The above examples are merely illustrative and should not be construed as limiting.

The purpose of the present application is to provide a composition for preventing and treating *M. hyorhinis* infection, which may include: (a) a fusion protein of C-terminus of DnaK - N-terminus of P72 - XylF of the *M*. *hyorhinis;* and (b) a pharmaceutically acceptable adjuvant. By expressing the fusion protein containing multiple antigens through an expression system (e.g., *E*. *coli*) to serve as the active ingredient of a vaccine, the vaccine production process and cost are significantly reduced, thereby optimizing the process proposed by the inventors in previous research and providing a greater advantage in the art.

**As** used herein, the term "fusion protein" refers to an amino acid sequence composed of multiple different proteins. The order of the proteins may vary without affecting the intended function of the fusion protein in the present application. For example, from N-terminus to C-terminus, it may be "N-terminus of P72 - C-terminus of DnaK - XylF"; or "XylF - N-terminus of P72 - C-terminus of DnaK", etc. In one embodiment, the fusion protein provided herein is a DnaK C-terminus - P72 N-terminus - XylF fusion protein, whose amino acid sequence from N-terminus to C-terminus is "C-terminus of DnaK", "N-terminus of P72", and "XylF". The above are merely examples and should not be construed as limiting.

The term "fusion protein of C-terminus of DnaK - N-terminus of P72 - XylF" as used herein may be represented as "MHY-30". If referring to the nucleotide sequence encoding the fusion protein, it may be represented as "nucleotide sequence of MHY-30". Similarly, if referring to the amino acid sequence of the fusion protein, it may be represented as "amino acid sequence of MHY-30".

In one embodiment, the concentration of the fusion protein in the composition may range from 50 to 500 µg/mL. In a preferred embodiment, the concentration of the fusion protein in the composition may be 100 µg/mL, based on the total volume of the composition. However, those skilled in the art will understand that the aforementioned concentrations may change due to intended use. For example, for ease of transport and storage, those skilled in the art may formulate the aforementioned composition with a high concentration of the active ingredient and then dilute it before actual use.

In this document, the terms "C-terminus of DnaK" and "N-terminus of P72" are used, where "N-terminus" and "C-terminus" refer to specific regions of an amino acid sequence. Those skilled in the art define a free α-amino group of the amino acid sequence as "N-terminus" and a free α-carboxyl group thereof as "C-terminus". The conventional order for writing amino acid sequences in this art is from N-terminus to C-terminus. Therefore, "N-terminus" can represent the beginning of a sequence, while "C-terminus" represents the end of a sequence.

For example, a protein with a full length of 266 amino acids may have an "N-terminus" comprising amino acids 1-50, 37-100 or 23-115, whereas the "C-terminus" may comprise amino acids 150-235, 122-198 or 178-234. The designation of positions by reference to the N-terminus or C-terminus serves only to generally indicate whether an amino acid sequence is located toward the "beginning" or the "end" of the protein and does not constitute a precise positional definition. Accordingly, detailed positional information shall be as defined in the present specification.

For example, as used herein, the term "C-terminus of DnaK" may include amino acids 291-597 of the DnaK protein (e.g., NCBI Reference Sequence WP_013302427.1); the term "N-terminus of P72" may include amino acids 26-265 of the P72 protein (e.g., NCBI Reference Sequence WP_014335516.1); and the term "XylF" may include amino acids 25-447 of the XylF protein (e.g., NCBI Reference Sequence WP_027332477.1). The protein sequences described in Taiwanese Patent No. TWI606839 are incorporated herein by reference in their entirety.

It is worth noting that the amino acid sequence used herein may not be the full length of the protein. Without changing the effect that the fusion protein is intended to achieve in the present application, the present application may include amino acid sequences of different lengths of the aforementioned protein.

In one embodiment, the C-terminus of DnaK of the fusion protein of the composition may include the sequence set forth in SEQ ID NO: 9; the N-terminus of P72 may include the sequence set forth in SEQ ID NO: 11; and XylF may include the sequence set forth in SEQ ID NO: 13.

The amino acid sequences provided in the present application may include amino acid residues that do not belong to the protein described herein. For example, amino acids 1-10 (MRGSHHHHHH) of the C-terminus of DnaK (SEQ ID NO: 9) may include a tag sequence used for protein purification, and amino acids 318-332 (EAAAKEAAAKEAAAK) may constitute a linker peptide, which has been found to enhance the yield of the fusion protein. Similar situations apply to amino acids 241-255 (EAAAKEAAAKEAAAK) of the N-terminus of P72 (SEQ ID NO: 11) and amino acids 424-429 (HHHHHH) of XylF (SEQ ID NO: 13). These amino acid sequences do not affect the effectiveness of the fusion protein in its intended use.

In one embodiment, the fusion protein of the composition may comprise the sequence set forth in SEQ ID NO: 7. In the fusion protein, individual protein domains may be separated by a small number of (e.g., one, two, or three) amino acids, which are restriction enzyme cleavage sites artificially added to the nucleotides encoding amino acids for the convenience of genetic engineering manipulations. For example, the *Kpn*I restriction enzyme cleavage site GGTACC encodes amino acids such as glycine (G) and threonine (T), which does not affect the effects of the fusion protein's intended use.

In one embodiment, the composition of the present invention may further include a pharmaceutically acceptable adjuvant and/or a pharmaceutically acceptable additive. The pharmaceutically acceptable adjuvant is used to increase the immunological effects of the active ingredient, maintain the stability of the active ingredient and enhance the safety of the composition for vaccine use. The pharmaceutically acceptable adjuvant may be, but is not limited to, a Freund's complete or incomplete adjuvant, alumina gel, a surfactant, an anionic polymer, a peptide, an oil emulsion or a combination thereof. The pharmaceutically acceptable additive may be, but is not limited to, a solvent, a stabilizer, a diluent, a preservative, an antimicrobial agent, an antifungal agent, an isotonic agent, an absorption delaying agent or a combination thereof.

To express the aforementioned fusion protein, the present application provides an expression vector, which may include: (a) a nucleotide sequence encoding a fusion protein of C-terminus of DnaK - N-terminus of P72 - XylF; (b) a replicon; and (c) an expression element, which may include a promoter, a terminator and a ribosome binding site.

**As** used herein, the term "expression vector" refers to a vector into which the nucleotide sequences provided herein can be inserted and that is capable of replicating in large quantities or directing the translation of the fusion protein in a host. Suitable expression vectors may include, but are not limited to, pET series vectors for *E. coli* expression systems, pPIC series vectors for *Pichia pastoris* expression systems, baculovirus transfer plasmids such as the pOET series for insect cell expression systems, pCMV vectors for mammalian cell expression systems, and shuttle vectors capable of replication and expression in multiple hosts. Any expression vector may be used as long as it allows insertion of the nucleotide sequence. Those skilled in the art will understand that when an expression vector is used to express the desired protein, the choice of vector may depend on factors such as the characteristics of the protein, desired yield, and cost. Accordingly, the embodiments provided herein are exemplary and are not intended to limit the scope of the present application.

**As** used herein, the term "replicon" refers to the region where DNA replication begins, which at least contains the origin of replication.

As used herein, the term "expression element" refers to the element required in an expression system to initiate the transcription and translation process. The aforementioned expression element should at least include a promoter, a terminator, and a ribosome binding site. Preferably, the expression element may additionally include an operator, enhancer sequences for transcription/translation, or a combination thereof. In a preferred embodiment, the aforementioned expression vector is used in an *E*. *coli* expression system, and the aforementioned expression element is recognizable by *E. coli.*

In one embodiment, the nucleotide sequence of the expression vector encoding the C-terminus of DnaK may encode the amino acid sequence set forth in SEQ ID NO: 9; the nucleotide sequence encoding the N-terminus of P72 may encode the amino acid sequence set forth in SEQ ID NO: 11; and the nucleotide sequence encoding XylF may encode the amino acid sequence set forth in SEQ ID NO: 13.

In one embodiment, the nucleotide sequence of the expression vector encoding the C-terminus of DnaK may include a nucleotide sequence set forth in SEQ ID NO: 8 or a reverse complementary sequence thereof; the nucleotide sequence encoding the N-terminus of P72 may include a nucleotide sequence set forth in SEQ ID NO: 10 or a reverse complementary sequence thereof; and the nucleotide sequence encoding the XylF may include a nucleotide sequence set forth in SEQ ID NO: 12 or a reverse complementary sequence thereof.

In an embodiment, the nucleotide sequence of the expression vector encoding the fusion protein may encode an amino acid sequence set forth in SEQ ID NO: 7. In an embodiment, the nucleotide sequence of the expression vector encoding the fusion protein may include a nucleotide sequence set forth in SEQ ID NO: 6 or a reverse complementary sequence thereof. In an embodiment, the expression vector may include a nucleotide sequence set forth in SEQ ID NO: 5.

As used herein, the term "a reverse complementary sequence of a sequence" refers to the general representation of nucleotide sequences in this field, from the 5' end to the 3' end, because most polymerases transcribe or translate nucleotides sequentially from the 5' end to the 3' end. Therefore, the nucleotide sequences encoding fusion proteins or proteins provided in the present application, such as SEQ ID NO: 6, 8, 10, 12, etc., are represented according to conventions in this field. However, since the expression vector is a double-stranded nucleotide sequence, in some cases, it is necessary to insert the sequence in a reverse complementary manner to match the transcription direction of the promoter. For example, in the embodiments provided in the present application, to match the transcription direction of the T7 promoter, the nucleotide sequences encoding fusion proteins are all inserted in a reverse complementary manner. The sequence and its reverse complementary sequence are complementary nucleotide chains to each other and therefore can be considered as the same sequence in this field.

In an embodiment, the fusion protein of the expression vector may further include a tag, which may include a histidine tag (His-tag), a glutathione S-transferase tag (GST-tag), a FLAG tag, a maltose binding protein-tag (MBP-tag), a hemagglutinin tag (HA-tag) or a combination thereof.

As used herein, the term "tag" refers to a nucleotide sequence inserted into an expression vector together with the nucleotide sequence encoding the fusion protein. When the fusion protein containing the tag is transcribed and translated, the purification process is performed by the affinity binding of the tag to a specific resin. For example, the histidine tag used in the present application consists of multiple repeating histidine, which exhibits a high affinity for the nickel ion affinity column, allowing for the separation and purification of the fusion protein.

As used herein, the term "affinity column" refers to a column containing a resin capable of developing an affinity with the tag, thereby enabling the separation of the protein. In addition to the nickel ion column described above, commonly used affinity columns in the art include glutathione affinity columns, Flag antibody affinity columns, maltose-binding protein affinity columns, and hemagglutinin (HA) affinity columns. Purification of the fusion protein can thus be achieved through the use of the affinity column.

In one embodiment, the expression vector may further include a selectable marker, such as a kanamycin-resistant gene, but is not limited thereto.

In one embodiment, the fusion protein of the expression vector may be expressed in *E*. *coli.*

In one embodiment, a method for producing a fusion protein of C-terminus of DnaK - N-terminus of P72 - XylF is provided, which may include transcribing the aforementioned nucleotide sequence in an expression system, followed by translation to obtain the fusion protein.

As used herein, the term "expression system" refers to the expression vector that enables protein expression and its compatible host. For example, the *E*. *coli* expression system provided in the present application. Other commonly used expression systems in the art may also be employed to produce the fusion protein provided herein, including the *Pichia pastoris* expression system, the insect cell expression system and the mammalian cell expression system.

In one embodiment, use of a fusion protein of C-terminus of DnaK - N-terminus of P72 - XylF in preparation of a composition for preventing and treating symptoms caused by *M. hyorhinis* infection is provided. Preferably, the symptoms caused by the *M. hyorhinis* infection include pleurisy, peritonitis, pericarditis, arthritis or a combination thereof.

The following are specific examples of the invention claimed in the present application and should not be used to limit the scope of the present application.

Example 1: A composition for preventing and treating *M. hyorhinis* infection, including:
(a) a fusion protein of C-terminus of DnaK - N-terminus of P72 - XylF of the *M*. *hyorhinis;* and
(b) a pharmaceutically acceptable adjuvant.

Example 2: The composition as described in the foregoing example, wherein a concentration of the fusion protein is 50 to 500 µg/mL.

Example 3: The composition as described in the foregoing examples, wherein the C-terminus of DnaK includes the sequence set forth in SEQ ID NO: 9; the N-terminus of P72 includes the sequence set forth in SEQ ID NO: 11; the XylF includes the sequence set forth in SEQ ID NO: 13.

Example 4: The composition as described in the foregoing examples, wherein the fusion protein includes the sequence set forth in SEQ ID NO: 7.

Example 5: The composition as described in the foregoing examples, wherein the pharmaceutically acceptable adjuvant is a Freund's complete or incomplete adjuvant, an alumina gel, a surfactant, an anionic polymer, a peptide, an oil emulsion or a combination thereof.

Example 6: The composition as described in the foregoing examples further includes a pharmaceutically acceptable additive.

Example 7: The composition as described in the foregoing examples, wherein the pharmaceutically acceptable additive includes the solvent, the stabilizer, the diluent, the preservative, the antimicrobial agent, the antifungal agent, the isotonic agent, the absorption delaying agent or a combination thereof.

Example 8: An expression vector for expressing the fusion protein as described in any one of the foregoing examples, including:
(a) the nucleotide sequence encoding the fusion protein of C-terminus of DnaK - N-terminus of P72 - XylF;
(b) the replicon; and
(c) the expression element including the promoter, the terminator and the ribosome binding site.

Example 9: The expression vector as described in the foregoing example, wherein the nucleotide sequence encoding the C-terminus of DnaK encodes the amino acid sequence set forth in SEQ ID NO: 9; the nucleotide sequence encoding the N-terminus of P72 encodes the amino acid sequence set forth in SEQ ID NO: 11; the nucleotide sequence encoding the XylF encodes the amino acid sequence set forth in SEQ ID NO: 13.

Example 10: The expression vector as described in the foregoing examples, wherein the nucleotide sequence encoding the C-terminus of DnaK includes the nucleotide sequence set forth in SEQ ID NO: 8 or its reverse complementary sequence; the nucleotide sequence encoding the N-terminus of P72 includes the nucleotide sequence set forth in SEQ ID NO: 10 or its reverse complementary sequence; and the nucleotide sequence encoding the XylF includes the nucleotide sequence set forth in SEQ ID NO: 12 or its reverse complementary sequence.

Example 11: The expression vector as described in the foregoing examples, wherein the nucleotide sequence encoding the fusion protein encodes the amino acid sequence set forth in SEQ ID NO: 7.

Example 12: The expression vector as described in the foregoing examples, wherein the nucleotide sequence encoding the fusion protein includes the nucleotide sequence set forth in SEQ ID NO: 6 or its reverse complementary sequence.

Example 13: The expression vector as described in the foregoing examples, wherein the expression vector includes the nucleotide sequence set forth in SEQ ID NO: 5.

Example 14: The expression vector as described in the foregoing examples, wherein the fusion protein further includes a tag, and the tag includes the His-tag, the GST-tag, the FLAG tag, the MBP-tag, the HA-tag or a combination thereof.

Example 15: The expression vector as described in the foregoing examples further includes a selectable marker, and the selectable marker includes the drug-resistant selectable marker, the non-drug-resistant selectable marker or a combination thereof.

Example 16: The expression vector as described in the foregoing examples, wherein the fusion protein is expressed in *E*. *coli.*

Example 17: A method for producing the fusion protein of C-terminus of DnaK - N-terminus of P72 - XylF includes transcribing the nucleotide sequence as described in the foregoing examples in the expression system, followed by translation to obtain the fusion protein.

Example 18: Use of the fusion protein of C-terminus of DnaK - N-terminus of P72 - XylF in preparation of the composition for preventing and treating symptoms caused by *M. hyorhinis* infection.

Example 19: The use as described in the foregoing examples, wherein the symptoms caused by the *M*. *hyorhinis* infection include pleurisy, peritonitis, pericarditis, arthritis or a combination thereof.

The materials, methods and examples in the present application are illustrative in nature and are not intended to limit the invention unless otherwise stated. Although the invention may be practiced or tested using similar or equivalent methods or materials described herein, those described herein are more suitable.

### Embodiment 1: Expression vector construction, fusion protein expression and solubility analysis

### 1.1. Expression vector construction

The DNA sequence of the DnaKC-P72N-XylF fusion protein, namely the MHY-30 DNA sequence, was synthesized by GeneDireX (USA) using codons optimized for *E. coli.* Using the plasmid containing the MHY-30 DNA sequence as a template, PCR was performed using the DNAKCHISNDEIF (SEQ ID NO: 1)/XYLHISSALR (SEQ ID NO: 2) primer combination in conjunction with the Q5^{®} High-Fidelity DNA polymerase reagent (NEB, USA). Each 100 µL PCR reaction mixture contained 1x Q5 Reaction buffer, 200 µM dNTPs, 0.5 µM amplification primer, 40 ng pUC57-MHY30, and 2 U Q5^{®} High-Fidelity DNA polymerase. The PCR reaction conditions are shown in Table 1. After the reaction, the expected DNA fragment size was confirmed by 0.7% (w/v) agarose gel electrophoresis, and the PCR products were recovered using the Plus Gel Eluted Kit (GMbiolab, Taiwan).

**Table 1: PCR reaction conditions**

| Steps | Number of cycles | Temperature (°C) | Time (sec) |
|---|---|---|---|
| 1 | 1 | 98 | 30 |
| | | 98 | 10 |
| 2 | 35 | 55 | 30 |
| | | 72 | 120 |
| 3 | 1 | 72 | 100 |

PCR products were cleaved with *Nde*I and *Sal*I and then recovered using a PCR Clean Up Kit (GMbiolab, Taiwan). The DNA fragments were then ligated into pET29a(+) (abbreviated as pET29a) (Merck Millipore, Germany) cleaved with the same restriction enzyme using T4 DNA ligase. The ligation product was transformed into ECOS^{™} 9-5 (Yeastern, Taiwan). After colony growth, colony polymerase chain reaction was performed using the T7 promoter primer (SEQ ID NO: 3)/T7 terminator primer (SEQ ID NO: 4) combined with DreamTaq DNA polymerase reagent (Thermo Fisher Scientific, USA) to select transformants that might contain insert DNA. After confirming the presence of insert DNA in the recombinant plasmids of the transformants using 0.7% (w/v) agarose gel electrophoresis, the plasmids were extracted from the transformants using the Plasmid Miniprep Purification Kit II (GMbiolab, Taiwan) for DNA sequencing. The plasmid with the correct DNA sequence was named pET29-MHY30.

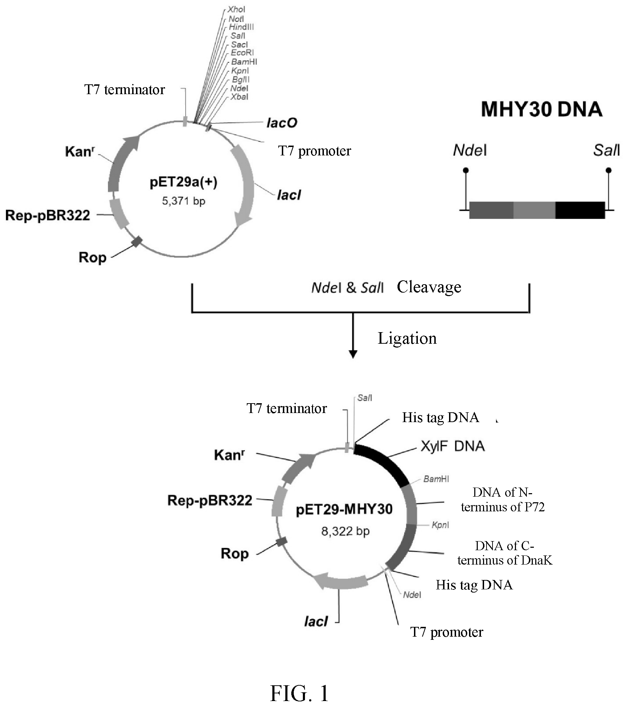

The construction process of pET29-MHY30 is shown in Figure 1; the sequence of the expression vector pET29-MHY30 (SEQ ID NO: 5), the MHY-30 DNA sequence in the expression vector (SEQ ID NO: 6), and the amino acid sequence of MHY-30 (SEQ ID NO: 7) are shown below.

Sequence of the expression vector pET29-MHY30 (SEQ ID NO: 5)

ATCCGGATATAGTTCCTCCTTTCAGCAAAAAACCCCTCAAGACCCGTTTA
GAGGCCCCAAGGGGTTATGCTAGTTATTGCTCAGCGGTGGCAGCAGCCAACTC
AGCTTCCTTTCGGGCTTTGTTAGCAGCCGGATCTCAGTGGTGGTGGTGGTGGT
GCTCGAGTGCGGCCGCAAGCTTGTCGACTTAATGATGATGATGATGATGCTGG
GTCACGCCATTGGTCGGAACGGTGCCGCTCGCGCTAACGCTAACATTCGCGC
TGGTGCTTTGCTGCGCGTTCGCCAGCGGGTTGTCAACGTTCTGCTTGGTCACA
ACCACCGGGTTCACCAGAATCGCCTTGATGTTTTTGTACGCGCCAACGCTACC
CGCTTTGTAGGTGGTATCGTCATAACGGAAGTGGATATCCTTGGTGTTTTCGTT
GTTTTTCAGCGCGTTCTCCACGTCTTCTTTGGTCGCGGTCGCGTTCGCTTTCTT

TTTGTTAATCAGGGTTTTCAGAACTTCCACCGCAACCTTGCCCAGGATAACGT
CCGGTTTGTAAATGGTCATGTTCTGCTCGCCGTTCTTGATCAGCTTTTTCGCGG
TGTCGTTGTAATCCTGACCGGTGATGAAAACCTTTTGCACATCGTAGTTGGTC
GCTTTCAGCTTGTCAACCGCCTGCTCCGCCATACCATCGTTCGGCGCCAGGAA
ACCCTTCAGAACGCTTTTCGGGATGGTCACGTTATCGTAGGTAACCGGCAGCA
CACCGTTCGGCACGCTGCTGTCACGATATTGGGTAAAGAATTGCTGAATACGC
TGGCCCGCATCACCGTAGTTCCAGCCCGGCACATAAACCGCGTTACCACGCG
GGCTCAGGTCAACCACTTTGCCGTTGCTGTTTTCCATCAGCTTTTTCAGGATCT
TCATCGCGCCGTTATAAAAGTACTGGCTGTTGTTATCGTCTTGGCTACCCGCA
ACCGCGTAGAAGCTCACGGTTTCTTGCGGCATGTGATCCTTCAGATAGTTCAG
CATCTCGGTTTCGTCCTTAAACGCACCATCGGTTTTACCCAGCAGACCCGCCG
CCAGGCTCAGGCCTTGCAGCTCGCCAACCTTTTCGTTGTCGTAGCTAACGTAC
CAATCGTAGCTGGTGCTGCCGGTGATCAGACGGTCGTACGCCACAATCGGGA
TGTTCTTCTCCGCAATGCTGTTAACCGCGGTGCCCACGCCACCGCCGTTCTCC
GGCGCAATGATGAAACCCTTCGGCGCCGGGTTCAGGTTCGCTTGCTGGGTCA
GCCAGTTGTTCTGCGCGTTTTGATCTTTGGTAATGGTGCTGATCGCACCTTCG
GTCTTGTCGATGTAGTTCAGGATATCCTTTTGCGCGTTGATCCAACGCGGGTT
ATCCGGGTCGGTCAGCGCAACACGAATGCTCTCGTTGGTCACCGCGTGGTCT
TTGGTCTGGCTCAGGCCGGTCGCGGTGGTGCCGCACGCAACAAAGCTGGATC
CCTTCGCAGCCGCTTCTTTAGCAGCCGCCTCTTTAGCAGCAGCCTCATCTTTC
ACATCTTTGCTCAGCTTGGTGGTGTCGATTTCGGTGTTATCGTAAATCTTCTGC
GCAAACTCGAACGCCTTTTTGATGTCGCTAAAGGTGCTATCGTTAACGGTATA
GCCGCTGAAACCGGTGTTTTTGCTGCTTTCTTTAATCTTGATCGCGCTGAACAT
GCTGCTGCTCGGGATGTGGTTGCCTTTACCCTCCGCTTCCTTCACCTTTTTCGC
AATGATGCTGTTCTCGTCAATGGTGCCACCACCCTGCTTGATGATGTCGAACA
GAACCTTCATGATATCCAGGTTGAACACAATACCATCGGTGTCGTTGATGTTG
TACGGCAGGCTCTTCAGGGTGGTCTGGCCCTCAACACCCAGCAGCTTGTTATA
GTTGTTAATGATTTTGGTCGGAAAGCTGTTCGGGTTAACGGTGCTGGTGCCCA
CGTCCAGCAGTTTATCGTAGCTGTTCGCCACATACGCGGTCGCCTTGTTGCCC
AGGATCAGGTTCCAATCGCTGCTTTGCTTGGTGCTCAGATCGCTGTCCAGGCT

GCTCAGCAGCTGACCCTCGCTGTTTTTGTGGGTAACGTCGCTGGTCAGCAGCT
CCACCGGGATAAAATCCGCGTCGTTTTTATGCTGTTCGTTGTAAATCTTGATGA
TCTCCTTCATGCCCAGCATCATCGGCCAATAGCTACCTTGGCTGGTCGCGAAC
TTGATGGTGGTCGGCTGACCGGTACCTTTCGCCGCCGCCTCTTTCGCCGCCGC
TTCTTTCGCCGCCGCCTCGTTTTCTTTCACTTCCGCTTCAATCGGTTGATCGTC
GCTGCTGGTGTCGCTGGTGTTCGCTTGCTGCGCCGCCGCCTGCGCAAACGCC
TGCGCCGCTTGTTCGATCTGATCCAGTTTGGTCTTCAGCTCTTCAATTTTCTCTT
CCTTAACCAGGTCCTTCAGCTCTTGGATCTGTTTCTCCAGGGTTTCTTTTTGCTT
CGCGTCCACTTTATCACCCTGGTCCTCCAGGCTCTTTTCCAGTTGGTTGATCAG
CGCTTCCGCACGCACGGTAACCTCAATCTTTTCCTTTTTCTTCGCATCCGCCTC
ACGGTTCTCTTCCGCCTCTTTCACCATACGTTCGATCTCTTCCTCGCTCAGGCT
GGTGGTGTTCTTAATGGTGATGGTCTGTTCCTTGTTGGTTTTCTTATCTTTCGCG
CTAACGGTGGTAATGCCGTTCACGTCGATGCTAAAGCTAACTTCGATCTGCGG
CACACCACGCGGCGCTTGCTCAATGCCGCTCAGGTTGAAACGACCCAGCAGC
TTGTTGTCCGCCGCCAGCTGACGCTCGCCTTGAACCACGCTGATGGTAACCTC
GGTTTGGTTGTTTTCCGCGGTGCTGAAAATCTGGCTTTTGGTCACCGGAATGG
TGGTGTTACGCGGAATCAGCGGGGTCGCAATGCCACCCAGGGTTTCGATACC
CAGGGTCAGCGGGGTAACGTCCAGCAGCAGCACATCGTTAATCTCGCCCGCC
AGAACGCCACCTTGAATCGCCGCACCGATCGCAACCACTTCGTCCGGGTTAA
TGCTACGGTTCGGTTTCTTGCCCAGGGTGTGCTCAATCATGGTCTGCACCGCC
GGAATACGGGTGCTACCGCCAACCAGCAGAACTTCGTCCAGGTCCTTCGCCT
CCAGCTTCGCCTCTTTCAGCGCATCCATAATCGGGTGGTGGTGGTGGTGC
GATCCTCTCATATGTATATCTCCTTCTTAAAGTTAAACAAAATTATTTCTAGAGGG
GAATTGTTATCCGCTCACAATTCCCCTATAGTGAGTCGTATTAATTTCGCGGGATC
GAGATCGATCTCGATCCTCTACGCCGGACGCATCGTGGCCGGCATCACCGGCG
CCACAGGTGCGGTTGCTGGCGCCTATATCGCCGACATCACCGATGGGGAAGAT
CGGGCTCGCCACTTCGGGCTCATGAGCGCTTGTTTCGGCGTGGGTATGGTGGC
AGGCCCCGTGGCCGGGGGACTGTTGGGCGCCATCTCCTTGCATGCACCATTCC
TTGCGGCGGCGGTGCTCAACGGCCTCAACCTACTACTGGGCTGCTTCCTAATG
CAGGAGTCGCATAAGGGAGAGCGTCGAGATCCCGGACACCATCGAATGGCGCA

AAACCTTTCGCGGTATGGCATGATAGCGCCCGGAAGAGAGTCAATTCAGGGTGG
TGAATGTGAAACCAGTAACGTTATACGATGTCGCAGAGTATGCCGGTGTCTCTT
ATCAGACCGTTTCCCGCGTGGTGAACCAGGCCAGCCACGTTTCTGCGAAAAC
GCGGGAAAAAGTGGAAGCGGCGATGGCGGAGCTGAATTACATTCCCAACCGC
GTGGCACAACAACTGGCGGGCAAACAGTCGTTGCTGATTGGCGTTGCCACCT
CCAGTCTGGCCCTGCACGCGCCGTCGCAAATTGTCGCGGCGATTAAATCTCGC
GCCGATCAACTGGGTGCCAGCGTGGTGGTGTCGATGGTAGAACGAAGCGGC
GTCGAAGCCTGTAAAGCGGCGGTGCACAATCTTCTCGCGCAACGCGTCAGTG
GGCTGATCATTAACTATCCGCTGGATGACCAGGATGCCATTGCTGTGGAAGCT
GCCTGCACTAATGTTCCGGCGTTATTTCTTGATGTCTCTGACCAGACACCCATC
AACAGTATTATTTTCTCCCATGAAGACGGTACGCGACTGGGCGTGGAGCATCT
GGTCGCATTGGGTCACCAGCAAATCGCGCTGTTAGCGGGCCCATTAAGTTCTG
TCTCGGCGCGTCTGCGTCTGGCTGGCTGGCATAAATATCTCACTCGCAATCAA
ATTCAGCCGATAGCGGAACGGGAAGGCGACTGGAGTGCCATGTCCGGTTTTC
AACAAACCATGCAAATGCTGAATGAGGGCATCGTTCCCACTGCGATGCTGGTT
GCCAACGATCAGATGGCGCTGGGCGCAATGCGCGCCATTACCGAGTCCGGGC
TGCGCGTTGGTGCGGACATCTCGGTAGTGGGATACGACGATACCGAAGACAG
CTCATGTTATATCCCGCCGTTAACCACCATCAAACAGGATTTTCGCCTGCTGGG
GCAAACCAGCGTGGACCGCTTGCTGCAACTCTCTCAGGGCCAGGCGGTGAA
GGGCAATCAGCTGTTGCCCGTCTCACTGGTGAAAAGAAAAACCACCCTGGCG
CCCAATACGCAAACCGCCTCTCCCCGCGCGTTGGCCGATTCATTAATGCAGCT
GGCACGACAGGTTTCCCGACTGGAAAGCGGGCAGTGAGCGCAACGCAATTAA
TGTAAGTTAGCTCACTCATTAGGCACCGGGATCTCGACCGATGCCCTTGAGAGC
CTTCAACCCAGTCAGCTCCTTCCGGTGGGCGCGGGGCATGACTATCGTCGCCG
CACTTATGACTGTCTTCTTTATCATGCAACTCGTAGGACAGGTGCCGGCAGCGCT
CTGGGTCATTTTCGGCGAGGACCGCTTTCGCTGGAGCGCGACGATGATCGGCC
TGTCGCTTGCGGTATTCGGAATCTTGCACGCCCTCGCTCAAGCCTTCGTCACTG
GTCCCGCCACCAAACGTTTCGGCGAGAAGCAGGCCATTATCGCCGGCATGGCG
GCCCCACGGGTGCGCATGATCGTGCTCCTGTCGTTGAGGACCCGGCTAGGCTG
GCGGGGTTGCCTTACTGGTTAGCAGAATGAATCACCGATACGCGAGCGAACGTG

AAGCGACTGCTGCTGCAAAACGTCTGCGACCTGAGCAACAACATGAATGGTCTT
CGGTTTCCGTGTTTCGTAAAGTCTGGAAACGCGGAAGTCAGCGCCCTGCACCA
TTATGTTCCGGATCTGCATCGCAGGATGCTGCTGGCTACCCTGTGGAACACCTA
CATCTGTATTAACGAAGCGCTGGCATTGACCCTGAGTGATTTTTCTCTGGTCCCG
CCGCATCCATACCGCCAGTTGTTTACCCTCACAACGTTCCAGTAACCGGGCATG
TTCATCATCAGTAACCCGTATCGTGAGCATCCTCTCTCGTTTCATCGGTATCATTA
CCCCCATGAACAGAAATCCCCCTTACACGGAGGCATCAGTGACCAAACAGGAAA
AAACCGCCCTTAACATGGCCCGCTTTATCAGAAGCCAGACATTAACGCTTCTGGA
GAAACTCAACGAGCTGGACGCGGATGAACAGGCAGACATCTGTGAATCGCTTC
ACGACCACGCTGATGAGCTTTACCGCAGCTGCCTCGCGCGTTTCGGTGATGAC
GGTGAAAACCTCTGACACATGCAGCTCCCGGAGACGGTCACAGCTTGTCTGTA
AGCGGATGCCGGGAGCAGACAAGCCCGTCAGGGCGCGTCAGCGGGTGTTGGC
GGGTGTCGGGGCGCAGCCATGACCCAGTCACGTAGCGATAGCGGAGTGTATAC
TGGCTTAACTATGCGGCATCAGAGCAGATTGTACTGAGAGTGCACCATATATGCG
GTGTGAAATACCGCACAGATGCGTAAGGAGAAAATACCGCATCAGGCGCTCTTC
CGCTTCCTCGCTCACTGACTCGCTGCGCTCGGTCGTTCGGCTGCGGCGAGCG
GTATCAGCTCACTCAAAGGCGGTAATACGGTTATCCACAGAATCAGGGGATAACG
CAGGAAAGAACATGTGAGCAAAAGGCCAGCAAAAGGCCAGGAACCGTAAAAAG
GCCGCGTTGCTGGCGTTTTTCCATAGGCTCCGCCCCCCTGACGAGCATCACAA
AAATCGACGCTCAAGTCAGAGGTGGCGAAACCCGACAGGACTATAAAGATACCA
GGCGTTTCCCCCTGGAAGCTCCCTCGTGCGCTCTCCTGTTCCGACCCTGCCGC
TTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCT
GTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACT
GGTAACAGGATTAGCAGAGCGAGGTATGTAGGCGGTGCTACAGAGTTCTTGAAG
TGGTGGCCTAACTACGGCTACACTAGAAGGACAGTATTTGGTATCTGCGCTCTG
CTGAAGCCAGTTACCTTCGGAAAAAGAGTTGGTAGCTCTTGATCCGGCAAACAA
ACCACCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGCTCAGT

GGAACGAAAACTCACGTTAAGGGATTTTGGTCATGAACAATAAAACTGTCTGCTT
ACATAAACAGTAATACAAGGGGTGTTATGAGCCATATTCAACGGGAAACGTCTT
GCTCTAGGCCGCGATTAAATTCCAACATGGATGCTGATTTATATGGGTATAAATG
GGCTCGCGATAATGTCGGGCAATCAGGTGCGACAATCTATCGATTGTATGGGA
AGCCCGATGCGCCAGAGTTGTTTCTGAAACATGGCAAAGGTAGCGTTGCCAAT
GATGTTACAGATGAGATGGTCAGACTAAACTGGCTGACGGAATTTATGCCTCTT
CCGACCATCAAGCATTTTATCCGTACTCCTGATGATGCATGGTTACTCACCACT
GCGATCCCCGGGAAAACAGCATTCCAGGTATTAGAAGAATATCCTGATTCAGG
TGAAAATATTGTTGATGCGCTGGCAGTGTTCCTGCGCCGGTTGCATTCGATTCC
TGTTTGTAATTGTCCTTTTAACAGCGATCGCGTATTTCGTCTCGCTCAGGCGCA
ATCACGAATGAATAACGGTTTGGTTGATGCGAGTGATTTTGATGACGAGCGTAA
TGGCTGGCCTGTTGAACAAGTCTGGAAAGAAATGCATAAACTTTTGCCATTCTC
ACCGGATTCAGTCGTCACTCATGGTGATTTCTCACTTGATAACCTTATTTTTGAC
GAGGGGAAATTAATAGGTTGTATTGATGTTGGACGAGTCGGAATCGCAGACCG
ATACCAGGATCTTGCCATCCTATGGAACTGCCTCGGTGAGTTTTCTCCTTCATTA
CAGAAACGGCTTTTTCAAAAATATGGTATTGATAATCCTGATATGAATAAATTGC
AGTTTCATTTGATGCTCGATGAGTTTTTCTAAGAATTAATTCATGAGCGGATACAT
ATTTGAATGTATTTAGAAAAATAAACAAATAGGGGTTCCGCGCACATTTCCCCGAA
AAGTGCCACCTGAAATTGTAAACGTTAATATTTTGTTAAAATTCGCGTTAAATTTTT
GTTAAATCAGCTCATTTTTTAACCAATAGGCCGAAATCGGCAAAATCCCTTATAAAT
CAAAAGAATAGACCGAGATAGGGTTGAGTGTTGTTCCAGTTTGGAACAAGAGTC
CACTATTAAAGAACGTGGACTCCAACGTCAAAGGGCGAAAAACCGTCTATCAGG
GCGATGGCCCACTACGTGAACCATCACCCTAATCAAGTTTTTTGGGGTCGAGGT
GCCGTAAAGCACTAAATCGGAACCCTAAAGGGAGCCCCCGATTTAGAGCTTGAC
GGGGAAAGCCGGCGAACGTGGCGAGAAAGGAAGGGAAGAAAGCGAAAGGAG
CGGGCGCTAGGGCGCTGGCAAGTGTAGCGGTCACGCTGCGCGTAACCACCAC
ACCCGCCGCGCTTAATGCGCCGCTACAGGGCGCGTCCCATTCGCCA

T7 promoter: 3317-3335 (boxed); MHY-30 gene: 179-3250 (bold); T7 terminator: 26-73 (boxed italic); lactose repressor gene: 3724-4808 (bold boxed); pBR322 replication region: 6238-6826 (gray background); kanamycin-resistant gene: 6948-7763 (bold italic).

MHY-30 DNA sequence in expression vector (SEQ ID NO: 6)

ATGAGAGGATCGCACCACCACCACCACCACCCGATTATGGATGCGCTG
AAAGAGGCGAAGCTGGAGGCGAAGGACCTGGACGAAGTTCTGCTGGTTGGCG
GTAGCACCCGTATTCCGGCGGTGCAGACCATGATTGAGCACACCCTGGGCAAG
AAACCGAACCGTAGCATTAACCCGGACGAAGTGGTTGCGATCGGTGCGGCGAT
TCAAGGTGGCGTTCTGGCGGGCGAGATTAACGATGTGCTGCTGCTGGACGTTA
CCCCGCTGACCCTGGGTATCGAAACCCTGGGTGGCATTGCGACCCCGCTGATT
CCGCGTAACACCACCATTCCGGTGACCAAAAGCCAGATTTTCAGCACCGCGGA
AAACAACCAAACCGAGGTTACCATCAGCGTGGTTCAAGGCGAGCGTCAGCTGG
CGGCGGACAACAAGCTGCTGGGTCGTTTCAACCTGAGCGGCATTGAGCAAGCG
CCGCGTGGTGTGCCGCAGATCGAAGTTAGCTTTAGCATCGACGTGAACGGCAT
TACCACCGTTAGCGCGAAAGATAAGAAAACCAACAAGGAACAGACCATCACCAT
TAAGAACACCACCAGCCTGAGCGAGGAAGAGATCGAACGTATGGTGAAAGAGG
CGGAAGAGAACCGTGAGGCGGATGCGAAGAAAAAGGAAAAGATTGAGGTTACC
GTGCGTGCGGAAGCGCTGATCAACCAACTGGAAAAGAGCCTGGAGGACCAGG
GTGATAAAGTGGACGCGAAGCAAAAAGAAACCCTGGAGAAACAGATCCAAGAG
CTGAAGGACCTGGTTAAGGAAGAGAAAATTGAAGAGCTGAAGACCAAACTGGA
TCAGATCGAACAAGCGGCGCAGGCGTTTGCGCAGGCGGCGGCGCAGCAAGCG
AACACCAGCGACACCAGCAGCGACGATCAACCGATTGAAGCGGAAGTGAAAGA
AAACGAGGCGGCGGCGAAAGAAGCGGCGGCGAAAGAGGCGGCGGCGAAAGG
TACCGGTCAGCCGACCACCATCAAGTTCGCGACCAGCCAAGGTAGCTATTGGC
CGATGATGCTGGGCATGAAGGAGATCATCAAGATTTACAACGAACAGCATAAAA
ACGACGCGGATTTTATCCCGGTGGAGCTGCTGACCAGCGACGTTACCCACAAA
AACAGCGAGGGTCAGCTGCTGAGCAGCCTGGACAGCGATCTGAGCACCAAGC
AAAGCAGCGATTGGAACCTGATCCTGGGCAACAAGGCGACCGCGTATGTGGCG
AACAGCTACGATAAACTGCTGGACGTGGGCACCAGCACCGTTAACCCGAACAG

CTTTCCGACCAAAATCATTAACAACTATAACAAGCTGCTGGGTGTTGAGGGCCA
GACCACCCTGAAGAGCCTGCCGTACAACATCAACGACACCGATGGTATTGTGTT
CAACCTGGATATCATGAAGGTTCTGTTCGACATCATCAAGCAGGGTGGTGGCAC
CATTGACGAGAACAGCATCATTGCGAAAAAGGTGAAGGAAGCGGAGGGTAAAG
GCAACCACATCCCGAGCAGCAGCATGTTCAGCGCGATCAAGATTAAAGAAAGC
AGCAAAAACACCGGTTTCAGCGGCTATACCGTTAACGATAGCACCTTTAGCGAC
ATCAAAAAGGCGTTCGAGTTTGCGCAGAAGATTTACGATAACACCGAAATCGAC
ACCACCAAGCTGAGCAAAGATGTGAAAGATGAGGCTGCTGCTAAAGAGGCGGC
TGCTAAAGAAGCGGCTGCGAAGGGATCCAGCTTTGTTGCGTGCGGCACCACC
GCGACCGGCCTGAGCCAGACCAAAGACCACGCGGTGACCAACGAGAGCATTC
GTGTTGCGCTGACCGACCCGGATAACCCGCGTTGGATCAACGCGCAAAAGGAT
ATCCTGAACTACATCGACAAGACCGAAGGTGCGATCAGCACCATTACCAAAGAT
CAAAACGCGCAGAACAACTGGCTGACCCAGCAAGCGAACCTGAACCCGGCGC
CGAAGGGTTTCATCATTGCGCCGGAGAACGGCGGTGGCGTGGGCACCGCGGT
TAACAGCATTGCGGAGAAGAACATCCCGATTGTGGCGTACGACCGTCTGATCA
CCGGCAGCACCAGCTACGATTGGTACGTTAGCTACGACAACGAAAAGGTTGGC
GAGCTGCAAGGCCTGAGCCTGGCGGCGGGTCTGCTGGGTAAAACCGATGGTG
CGTTTAAGGACGAAACCGAGATGCTGAACTATCTGAAGGATCACATGCCGCAA
GAAACCGTGAGCTTCTACGCGGTTGCGGGTAGCCAAGACGATAACAACAGCCA
GTACTTTTATAACGGCGCGATGAAGATCCTGAAAAAGCTGATGGAAAACAGCAA
CGGCAAAGTGGTTGACCTGAGCCCGCGTGGTAACGCGGTTTATGTGCCGGGCT
GGAACTACGGTGATGCGGGCCAGCGTATTCAGCAATTCTTTACCCAATATCGTG
ACAGCAGCGTGCCGAACGGTGTGCTGCCGGTTACCTACGATAACGTGACCATC
CCGAAAAGCGTTCTGAAGGGTTTCCTGGCGCCGAACGATGGTATGGCGGAGCA
GGCGGTTGACAAGCTGAAAGCGACCAACTACGATGTGCAAAAGGTTTTCATCAC
CGGTCAGGATTACAACGACACCGCGAAAAAGCTGATCAAGAACGGCGAGCAGA
ACATGACCATTTACAAACCGGACGTTATCCTGGGCAAGGTTGCGGTGGAAGTTC
TGAAAACCCTGATTAACAAAAAGAAAGCGAACGCGACCGCGACCAAAGAAGAC
GTGGAGAACGCGCTGAAAAACAACGAAAACACCAAGGATATCCACTTCCGTTAT
GACGATACCACCTACAAAGCGGGTAGCGTTGGCGCGTACAAAAACATCAAGGC

GATTCTGGTGAACCCGGTGGTTGTGACCAAGCAGAACGTTGACAACCCGCTGG
CGAACGCGCAGCAAAGCACCAGCGCGAATGTTAGCGTTAGCGCGAGCGGCAC
CGTTCCGACCAATGGCGTGACCCAGCATCATCATCATCATCATTAA

5' end ~ *Kpn*I (GGTACC): C-terminal DNA sequence of DnaK; *Kpn*I (GGTACC) ~ *Bam*HI (GGATCC): N-terminal DNA sequence of P72; *Bam*HI (GGATCC) ~ 3' end: XylF DNA sequence, Termination code TAA does not produce amino acids; bold text: His-tag DNA sequence; bold boxed text: cleavage site of restriction enzyme.

Amino acid sequence of MHY-30 (SEQ ID NO: 7)

MRGSHHHHHHPIMDALKEAKLEAKDLDEVLLVGGSTRIPAVQTMIEHTLG
KKPNRSINPDEVVAIGAAIQGGVLAGEINDVLLLDVTPLTLGIETLGGIATPLIPRNTT
IPVTKSQIFSTAENNQTEVTISVVQGERQLAADNKLLGRFNLSGIEQAPRGVPQIEV
SFSIDVNGITTVSAKDKKTNKEQTITIKNTTSLSEEEIERMVKEAEENREADAKKKE
KIEVTVRAEALINQLEKSLEDQGDKVDAKQKETLEKQIQELKDLVKEEKIEELKTKL
DQIEQAAQAFAQAAAQQANTSDTSSDDQPIEAEVKENEAAAKEAAAKEAAAKGT
GQPTTIKFATSQGSYWPMMLGMKEIIKIYNEQHKNDADFIPVELLTSDVTHKNSEGQ
LLSSLDSDLSTKQSSDWNLILGNKATAYVANSYDKLLDVGTSTVNPNSFPTKIINNY
NKLLGVEGQTTLKSLPYNINDTDGIVFNLDIMKVLFDIIKQGGGTIDENSIIAKKVKEAE
GKGNHIPSSSMFSAIKIKESSKNTGFSGYTVNDSTFSDIKKAFEFAQKIYDNTEIDTTK
LSKDVKDEAAAKEAAAKEAAAKGSSFVACGTTATGLSQTKDHAVTNESIRVALTDP
DNPRWINAQKDILNYIDKTEGAISTITKDQNAQNNWLTQQANLNPAPKGFIIAPENG
GGVGTAVNSIAEKNIPIVAYDRLITGSTSYDWYVSYDNEKVGELQGLSLAAGLLGK
TDGAFKDETEMLNYLKDHMPQETVSFYAVAGSQDDNNSQYFYNGAMKILKKLME
NSNGKVVDLSPRGNAVYVPGWNYGDAGQRIQQFFTQYRDSSVPNGVLPVTYDNV
TIPKSVLKGFLAPNDGMAEQAVDKLKATNYDVQKVFITGQDYNDTAKKLIKNGEQ
NMTIYKPDVILGKVAVEVLKTLINKKKANATATKEDVENALKNNENTKDIHFRYDD
TTYKAGSVGAYKNIKAILVNPVVVTKQNVDNPLANAQQSTSANVSVSASGTVPTN
GVTQHHHHHH

Bold text: C-terminus of DnaK; Italic text: N-terminus of P72; Bold italic text: XylF; Gray background: Histidine tag; Boxed text: Amino acid sequence generated by restriction enzyme cleavage site.

### 1.2. Fusion protein expression

The expression vector was transformed into *E*. *coli* BL21(DE3). A single colony was inoculated into 5 mL of LB medium containing 30 µg/mL kanamycin and cultured at 37°C and 180 rpm. After overnight culture, 50 µL of the afore-cultured bacterial solution was added to 5 mL of LB medium containing 30 µg/mL kanamycin and cultured with shaking (37°C, 180 rpm) until the OD₆₀₀ reached approximately 0.4 to 0.6. Afterwards, 0.1 mM isopropyl-β-D-thiogalactoside (IPTG) was added, and the induction expression of the fusion protein was performed at 25°C. After 4 hours of induction, 1 mL of the bacterial culture with OD₆₀₀=2 was centrifuged (20,630 ×g, 5 min, 4°C), and the bacterial cell fraction was collected.

### 1.3. Solubility analysis

The collected bacterial cells were suspended in 500 µL of SET buffer (50 mM glucose, 10 mM EDTA, 25 mM Tris, pH 8.0). The cells were lysed using an ultrasonic homogenizer (Sonics VCX-750, USA) under the following conditions: amplitude 20%; intermittent treatment (9 seconds of treatment followed by a 9-second pause); total treatment time being 2 minutes.

100 µL of the bacterial lysate was mixed with 25 µL of 5×Sample buffer to obtain the total cell lysate. 100 µL of the bacterial lysate was centrifuged (20, 630 × g, 10 minutes, 4°C), and the supernatant was mixed with 25 µL of 5×Sample buffer to obtain the soluble protein. 25 µL each of the total cell lysate and the soluble protein sample were heated at 100°C for 10 minutes, and then the protein solubility was analyzed by protein electrophoresis and immunoblotting.

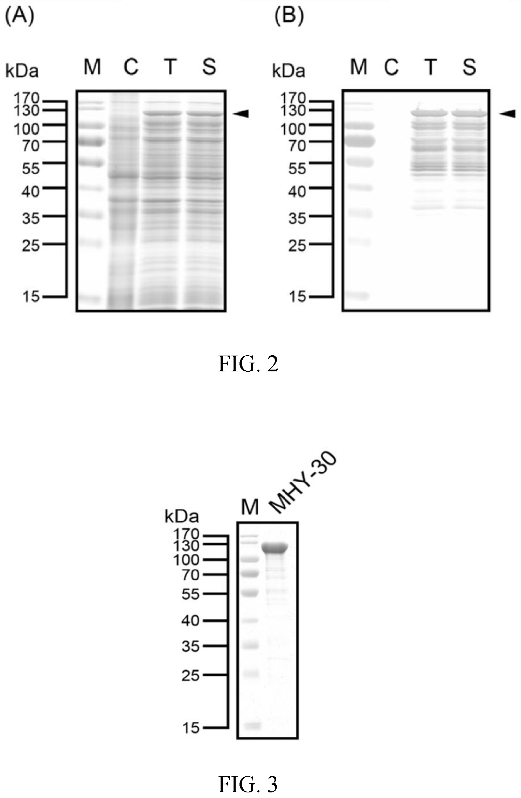

The results (Figure 2) showed that the fusion protein could be expressed in *E*. *coli* and was mainly a soluble protein.

### Embodiment 2: Expression and purification of fusion protein

### 2.1. Expression of fusion protein

A single colony was inoculated into 12 mL of LB medium containing 30 µg/mL kanamycin and cultured at 37°C and 180 rpm. After overnight culture, 10 mL of the afore-cultured bacterial solution was added to 1 L of LB medium containing 30 µg/mL kanamycin and cultured with shaking (37°C, 180 rpm) until the OD₆₀₀ reached approximately 0.4 to 0.6. Afterwards, 0.1 mM isopropyl-β-D-thiogalactoside (IPTG) was added, and the induction expression of the fusion protein was performed at 25°C. After 4 hours of induction, the bacterial cell fraction was collected by centrifugation (10,000 ×g, 10 min, 4°C).

The bacterial cells cultured in a 2 L shake flask were mixed with 96 mL of Lysis buffer (20 mM sodium phosphate, 500 mM NaCl, 1% (v/v) Triton X-100, pH 7.4). The cells were disrupted using a high-pressure cell disruptor at 2,000 bar. The cell disruption solution was centrifuged (20,630 × g, 4°C, 30 min), followed by collection of the supernatant. The cell fragments were removed from the supernatant using a 0.22 µm polyethersulfone (PES) filter membrane and a peristaltic pump.

### 2.2. Purification of fusion protein

Purification of the fusion protein was performed using immobilized-metal ion affinity chromatography based on the ability of the histidine tag on the fusion protein to form coordinate covalent bonds with nickel or cobalt ions. The purification of the fusion protein was carried out using a protein liquid chromatography system ÄKTA prime plus (GE Healthcare, Sweden) equipped with a 5 mL HiTrap Excel column (GE Healthcare, Sweden). First, the column was equilibrated with 25 mL of equilibration buffer (20 mM sodium phosphate, 500 mM NaCl, pH 7.4), after which the filtered supernatant was injected into the column. After the sample injection was completed, non-specifically bound proteins were removed by washing the column with 100 mL of Wash Buffer A (20 mM sodium phosphate, 500 mM NaCl, 30 mM imidazole, Triton X-114, pH 7.4), followed by an additional wash with 100 mL of Wash Buffer B (20 mM sodium phosphate, 500 mM NaCl, 30 mM imidazole, pH 7.4) to remove the non-specifically bound proteins. Subsequently, the fusion protein on the resin was eluted using a mixture of 100 mL wash buffer B and elution buffer (20 mM sodium phosphate, 500 mM NaCl, 250 mM imidazole, pH 7.4) with 30 mM to 250 mM imidazole. Finally, the column was eluted with 50 mL of the elution buffer. The protein purification was monitored by protein electrophoresis. The concentration of the purified protein was determined using the Protein Assay Kit II (Bio-Rad, USA), and the purity of the purified protein was analyzed using ImageQuant software.

Taking advantage of the property that fusion proteins, carrying histidine tags, can form coordinate covalent bonds with nickel or cobalt ions, an immobilized metal ion affinity chromatography method was used to purify intracellular soluble fusion proteins from *E*. *coli.* The results showed that the fusion proteins could be purified using a HiTrap^{™} Ni excel column (Figure 3).

### Embodiment 3: Preparation of subunit vaccine and swine immune challenge test

### 3.1. Preparation of subunit vaccine

A subunit vaccine was prepared using the fusion protein described in Embodiment 2 as the active ingredient to evaluate its immunoprotective effects *in vivo* in a porcine model. The subunit vaccine could be prepared by mixing the fusion protein with a suitable adjuvant uniformly. Each dose of the vaccine had a total volume of 2 mL and contained 200 µg of the fusion protein.

### 3.2. Swine immune challenge test

Twelve pigs from a commercial pig farm were randomly divided into groups of six pigs each before the experiment. The immunization group received a single dose (2 mL) of vaccine via intramuscular injection in the neck at 1 and 3 weeks of age, while the control group received an equal volume of physiological saline via intramuscular injection in the neck at the same time. All experimental pigs were challenged at 8 weeks of age with a culture of *M*. *hyorhinis* isolated from the wild. At 3 weeks post-challenge (11 weeks of age), necropsy and pathological examination were performed to calculate the percentage of lesions, such as peritonitis, pleurisy, pericarditis, and joint swelling. Gross lesion scoring was performed according to the method described by Magnusson et al. (Vet. Immunol. Immunopathol., 61: 83-96, 1998). Clinical symptoms were observed daily, and pigs were weighed regularly to assess vaccine safety in the target animals. Serum samples were collected regularly for antibody testing to monitor changes in serum antibodies after vaccination.

The results of the swine immunization challenge test showed that the immunized pigs had good spirits and activity levels after immunization, and no adverse reactions such as redness, swelling or inflammation occurred at the immunization site, indicating that the vaccine has high safety. Lesion analysis results showed that the vaccine could reduce the severity of peritonitis, pericarditis and arthritis in pigs (Table 2), indicating that the vaccine can induce an immune protective response and can be used to prevent and treat the *M*. *hyorhinis* infection.

**Table 2 Gross lesion scoring results of swine immune challenge test.**

| Group | Pig No. | Lesion Scoring | | | | | |
|---|---|---|---|---|---|---|---|
| | | Pleurisy | Peritonitis | Pericarditis | Arthritis | Total Score | Total |
| Immunization group | 2106 | 1 | 0 | 0 | 1 | 2 | 12 |
| | 2107 | 0 | 0 | 0 | 0 | 0 | |
| | 2110 | 0 | 0 | 0 | 2 | 2 | |
| | 2111 | 0 | 0 | 0 | 1 | 1 | |
| | 2112 | 0 | 0 | 0 | 0 | 0 | |
| | 2113 | 0 | 3 | 3 | 1 | 7 | |
| Mean | | 0.2 | 0.5 | 0.5 | 0.8 | 2.0 | 4 |
| Control group | 4004 | 1 | 0 | 1 | 3 | 5 | 33 |
| | 4005 | 3 | 0 | 1 | 3 | 7 | |
| | 4006 | 0 | 0 | 1 | 1 | 2 | |
| | 4008 | 2 | 1 | 1 | 3 | 7 | |
| | 4009 | 3 | 2 | 3 | 1 | 9 | |
| | 4010 | 0 | 0 | 0 | 3 | 3 | |
| Mean | | 1.5 | 0.5 | 1.2 | 2.3 | 5.5 | 11 |

## Claims

1. A composition for preventing and treating *Mycoplasma hyorhinis* (*M. hyorhinis*) infection, comprising:
a fusion protein of C-terminus of DnaK - N-terminus of P72 - XylF of the *M*. *hyorhinis;* and
a pharmaceutically acceptable adjuvant.

2. The composition according to claim 1, wherein a concentration of the fusion protein is 50 to 500 µg/mL.

3. The composition according to claim 1, wherein the C-terminus of DnaK comprises a sequence set forth in SEQ ID NO: 9; the N-terminus of P72 comprises a sequence set forth in SEQ ID NO: 11; or the XylF comprises a sequence set forth in SEQ ID NO: 13.

4. The composition according to claim 3, wherein the fusion protein comprises a sequence set forth in SEQ ID NO: 7.

5. The composition according to claim 1, wherein the pharmaceutically acceptable adjuvant is a Freund's complete or incomplete adjuvant, an alumina gel, a surfactant, an anionic polymer, a peptide, an oil emulsion or a combination thereof.

6. The composition according to claim 1, further comprising a pharmaceutically acceptable additive.

7. The composition according to claim 6, wherein the pharmaceutically acceptable additive comprises a solvent, a stabilizer, a diluent, a preservative, an antimicrobial agent, an antifungal agent, an isotonic agent, an absorption delaying agent or a combination thereof.

8. An expression vector for expressing the fusion protein according to any one of claims 1 to 7, comprising:
a nucleotide sequence encoding a fusion protein of C-terminus of DnaK - N-terminus of P72 - XylF;
a replicon; and
an expression element, comprising a promoter, a terminator and a ribosome binding site.

9. The expression vector according to claim 8, wherein a nucleotide sequence encoding the C-terminus of DnaK encodes an amino acid sequence set forth in SEQ ID NO: 9; a nucleotide sequence encoding the N-terminus of P72 encodes an amino acid sequence set forth in SEQ ID NO: 11; and a nucleotide sequence encoding the XylF encodes an amino acid sequence set forth in SEQ ID NO: 13.

10. The expression vector according to claim 8, wherein the nucleotide sequence encoding the C-terminus of DnaK comprises a nucleotide sequence set forth in SEQ ID NO: 8 or a reverse complementary sequence thereof; the nucleotide sequence encoding the N-terminus of P72 comprises a nucleotide sequence set forth in SEQ ID NO: 10 or a reverse complementary sequence thereof; and the nucleotide sequence encoding the XylF comprises a nucleotide sequence set forth in SEQ ID NO: 12 or a reverse complementary sequence thereof.

11. The expression vector according to claim 8, wherein the nucleotide sequence encoding the fusion protein encodes an amino acid sequence set forth in SEQ ID NO: 7.

12. The expression vector according to claim 11, wherein the nucleotide sequence encoding the fusion protein comprises a nucleotide sequence set forth in SEQ ID NO: 6 or a reverse complementary sequence thereof.

13. The expression vector according to claim 8, wherein the expression vector comprises a nucleotide sequence set forth in SEQ ID NO: 5.

14. The expression vector according to claim 8, wherein the fusion protein further comprises a tag, which comprises a histidine tag (His-tag), a glutathione S-transferase tag (GST-tag), a FLAG tag, a maltose binding protein-tag (MBP-tag), a hemagglutinin tag (HA-tag) or a combination thereof.

15. The expression vector according to claim 8, further comprising a selectable marker, which comprises a drug-resistant selectable marker, a non-drug-resistant selectable marker or a combination thereof.

16. The expression vector according to claim 8, wherein the fusion protein is expressed in *E*. *coli.*

17. A method for producing a fusion protein of C-terminus of DnaK - N-terminus of P72 - XylF, comprising transcribing the nucleotide sequence according to any one of claims 8 to 16 in an expression system, followed by translation to obtain the fusion protein.

18. Use of a fusion protein of C-terminus of DnaK - N-terminus of P72 - XyIF in preparation of a composition for preventing and treating symptoms caused by *M. hyorhinis* infection.

19. The use according to claim 18, wherein the symptoms caused by *M. hyorhinis* infection comprises pleurisy, peritonitis, pericarditis, arthritis or a combination thereof.
